# EUROPEAN PATENT APPLICATION

(11) **EP 4 059 958 A1**
(43) Date of publication of application: **21.09.2022**
(21) Application number: 20887732.4
(22) Date of filing: 11.11.2020
(51) Int. Cl.: C07K 16/10, C12N 15/13, G01N 33/543, G01N 33/569, C12P 21/08

(54) **ANTI?RS VIRUS N PROTEIN?RECOGNIZING ANTIBODY, IMMUNOASSAY METHOD AND IMMUNOASSAY INSTRUMENT USING ANTI?RS VIRUS N PROTEIN?RECOGNIZING ANTIBODY**

(30) Priority: 12.11.2019 JP 2019204604
(71) Applicant: Denka Company Limited, Tokyo 103-8338 (JP)
(72) Inventor: KUWAHARA Miwa, Gosen-shi, Niigata 959-1695 (JP)
(74) Representative: Wohlfahrt, Jan Günther
(86) International application number: PCT/JP2020/042063
(87) International publication number: WO 2021/095762

(57) **Abstract**

Provided are an anti-RS virus antibody with high sensitivity and a test reagent using the antibody.

An anti-RS virus N protein monoclonal antibody or an antigen-binding fragment thereof, which is characterized in that it reacts with peptides consisting of the amino acid sequences as set forth in, at least, SEQ ID NOs: 30, 39, 40, 69, 70, 74, 75, 83, 84, 100, 101, and 113, among 127 peptide spots produced based on the amino acid sequence of the N protein of an RS virus.

## Description

### Technical Field

The present invention relates to an immunoassay method and an immunoassay apparatus, which are used in the immunological measurement of an RS virus, and an anti-RS virus antibody used therefor.

### Background Art

Since a monoclonal antibody recognizes only a specific antigen, the monoclonal antibody is widely used in detection of the specific antigen. In the envelope of an RS virus (respiratory syncytial virus), a G protein as a glycoprotein and an F protein associated with cell fusion are present (Non Patent Literature 1). It has been known that there is a large difference in the amino acid sequence of the G protein between the subtypes (type A and type B) of the RS virus (Non Patent Literature 1). On the other hand, it has been known that there is a small difference in the amino acid sequence of the F protein between the subtypes (type A and type B) of the RS virus, and a major number of RS virus-testing kits have been detection of the F protein. However, it has been extremely difficult to develop an RS virus-testing kit exhibiting a practical positive detection rate, which is capable of detecting clinically isolated strains without omission, only by enhancing the affinity of an antibody for the F protein (Patent Literature 1).

In addition, the N protein of the RS virus is also referred to as a "nucleoprotein," and this N protein consists of 391 amino acid residues. The N protein is a structural component of a ribonucleoprotein complex called a "nucleocapsid," and surrounds the genomic RNA of the RS virus to form a spiral structure. It is known that a difference in the amino acid sequence of the N protein is smallest between the subtypes (type A and type B) of the RS virus. However, it has been extremely difficult to detect clinically isolated strains without omission, only by using the antibody reacting against the N protein. Thus, in order to develop an RS virus-testing kit capable of detecting clinically isolated strains without omission, it has been necessary to mix the antibody reacting against the F protein with the antibody reacting against the N protein (Patent Literature 1).

### Citation List

### Patent Literature

Patent Literature 1: Japanese Patent No. 6454274

### Non Patent Literature

Non Patent Literature 1: Collins PL and Karron RA. Respiratory syncytial virus. In: Knipe DM et al., eds. Fields Virology, 6th ed. Lippincott Williams & Wilkins, Philadelphia: pp. 1086-1123 (2013).

### Summary of Invention

### Technical Problem

At present, test reagents using various types of anti-RS virus antibodies have been commercially available. However, when the RS virus has been detected using these conventional RS virus test reagents, the sensitivity has not been sufficient.

Under the above-described current circumstances, it is an object of the present invention to provide an anti-RS virus antibody with high sensitivity, and a test reagent using the antibody.

### Solution to Problem

The present inventors have found that, in order to enhance the sensitivity of an immunochromatography device by efficiently capture an RS virus contained in a specimen at a determination site, an antibody reacting against the N protein (nucleoprotein) of the RS virus is used, so that sensitivity to detect the RS virus can be improved, thereby completing the present invention.

Specifically, the present invention is as follows.
[1] An anti-RS virus N protein monoclonal antibody or an antigen-binding fragment thereof, which is characterized in that it reacts with peptides consisting of the amino acid sequences as set forth in, at least, SEQ ID NOs: 30, 39, 40, 69, 70, 74, 75, 83, 84, 100, 101, and 113, among 127 peptide spots produced based on the amino acid sequence of the N protein of an RS virus.
[2] The anti-RS virus N protein monoclonal antibody or an antigen-binding fragment thereof according to the above [1], wherein the antigen-binding fragment is a peptide fragment selected from the group consisting of Fab, Fab', F(ab')2, a single-chain antibody (scFv), dsFv, a diabody, and a minibody.
[3] An RS virus detection reagent, comprising the monoclonal antibody according to the above [1] or [2], or an antigen-binding fragment thereof.
[4] The RS virus detection reagent according to the above [3], which is an immunochromatography reagent.
[5] An RS virus detection kit, comprising the monoclonal antibody according to the above [1] or [2], or an antigen-binding fragment thereof.
[6] A method for detecting an RS virus, using the monoclonal antibody according to the above [1] or [2], or an antigen-binding fragment thereof, according to an immunological detection method.
[7] The method according to the above [6], which is an immunochromatography.

The present description includes the contents as disclosed in Japanese Patent Application No. 2019-204604, which is a priority document of the present application.

### Advantageous Effects of Invention

According to the method of the present invention, since an anti-RS virus N protein antibody is used in immunoassay, high sensitivity can be obtained. In addition, according to the present invention, an immunoassay apparatus and a monoclonal antibody, which are used in a novel detection method of the present invention, are provided.

### Brief Description of Drawings

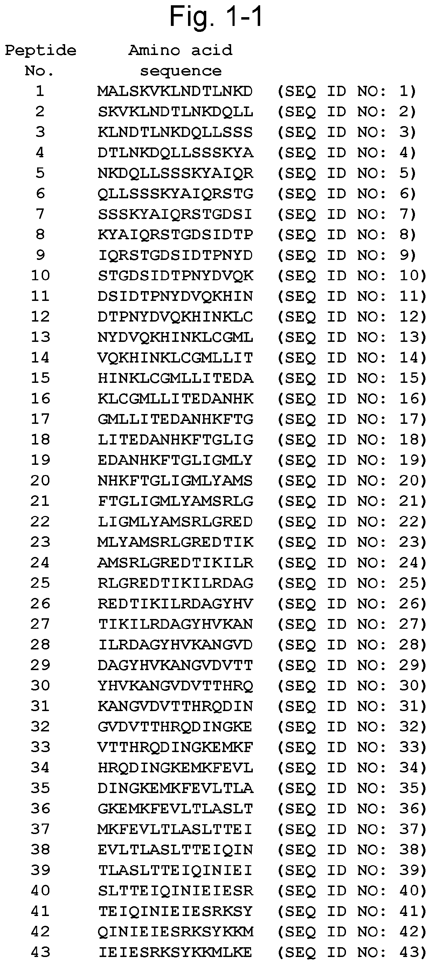

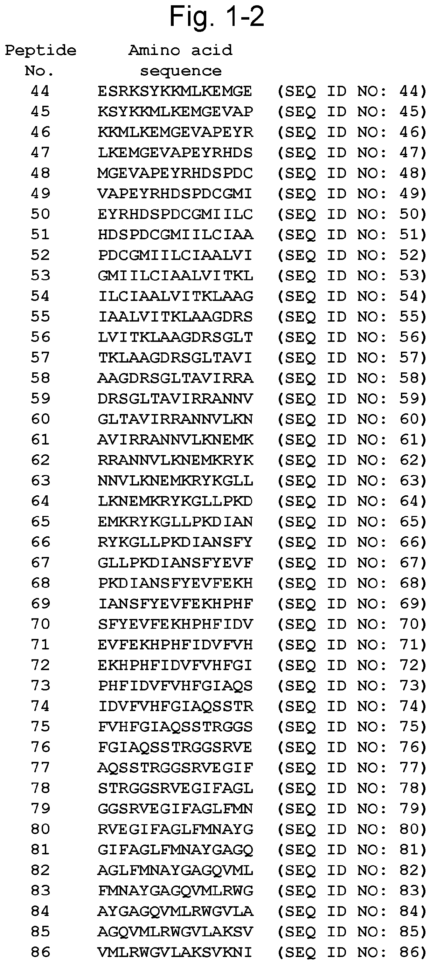

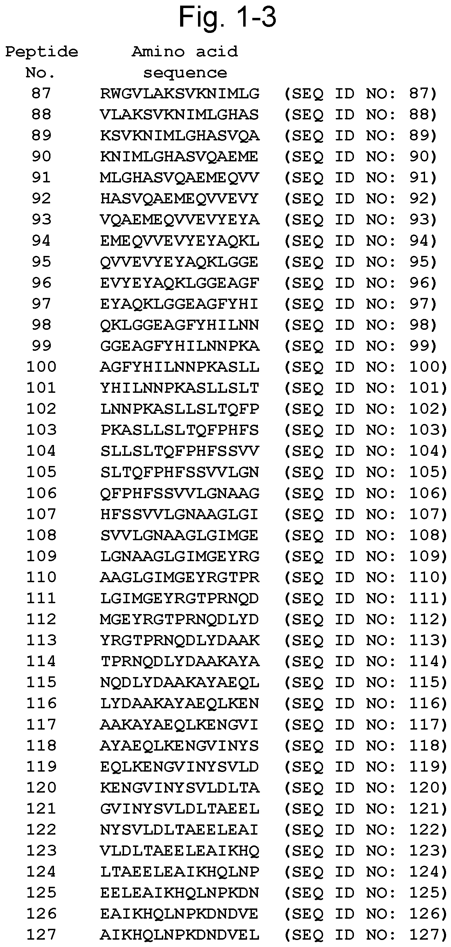

[Figure 1-1] Figure 1-1 is a view showing the amino acid sequences of 127 peptides, which are formed by shifting by 3 amino acids of a partial peptide consisting of 15 amino acids from the amino acid sequence (SEQ ID NO: 128) consisting of 391 amino acids of the N protein of an RS virus (Peptide Nos. 1 to 43).
[Figure 1-2] Figure 1-2 is a view showing the amino acid sequences of 127 peptides, which are formed by shifting by 3 amino acids of a partial peptide consisting of 15 amino acids from the amino acid sequence (SEQ ID NO: 128) consisting of 391 amino acids of the N protein of an RS virus (Peptide Nos. 44 to 86).
[Figure 1-3] Figure 1-3 is a view showing the amino acid sequences of 127 peptides, which are formed by shifting by 3 amino acids of a partial peptide consisting of 15 amino acids from the amino acid sequence (SEQ ID NO: 128) consisting of 391 amino acids of the N protein of an RS virus (Peptide Nos. 87 to 127).

### Description of Embodiments

The test subject detected by the method of the present invention is an RS virus, and a monoclonal antibody that recognizes an N protein as an antigen is used.

According to the method of the present invention, an immunoassay is carried out using a monoclonal antibody that recognizes an N protein as an antigen, or an antigen-binding fragment thereof. Herein, the term "to recognize" means to specifically react, namely, to perform an antigen-antibody reaction. The term "specific" means that the aforementioned antibody does not cause an antigen-antibody reaction, at a detectable level, with the protein component of the antigen in a liquid system in which the protein is mixed with the antibody, or that even if the aforementioned antibody causes a certain binding reaction or association reaction, the level of the reaction is clearly weaker than the antigen-antibody reaction of the antibody with the antigen.

An antigen-binding fragment formed by separating only an antigen-binding site from the monoclonal antibody of the present invention can also be used in the method of the present invention. That is to say, when fragments having specific antigen-binding ability (antigen-binding fragments), which are produced by known methods and bind to the N protein of the RS virus, such as Fab, Fab', F(ab')2, a single-chain antibody (scFv), dsFv, a diabody, and a minibody, are used, these fragments are encompassed in the present monoclonal antibody and are included in the scope of the present invention. In addition, the class of the monoclonal antibody is not limited to IgG, and may also be IgM or IgY.

The monoclonal antibody used in the method of the present invention can be obtained by immunizing an animal to be immunized with an antigen containing a complex or extract of interest, or an antigen or a partial peptide thereof according to a known immunological method, and then producing hybridomas using the cells of the immunized animal. The length of the peptide used in immunization is not particularly limited, and a peptide consisting of preferably 5 or more amino acids, and more preferably 10 or more amino acids can be used as an immunogen. Such an immunogen can be obtained from a culture solution, but it can also be obtained by incorporating DNA encoding any given antigen into a plasmid vector and then introducing the vector into host cells, so that it can be expressed therein. Any given antigen used as an immunogen or a partial peptide thereof is expressed as a fusion protein that is fused with any of the below-exemplified proteins, and it can be used as an immunogen, after purification or in an unpurified state. For the production of fusion proteins, there can be utilized glutathione S-transferase (GST), maltose-binding protein (MBP), thioredoxin (TRX), Nus tag, S tag, HSV tag, FRAG tag, polyhistidine tag, etc., which are commonly used as "protein expression/purification tags" by those skilled in the art. The fusion protein fused with any of these proteins is preferably used as an immunogen, after any given antigen or a partial peptide portion thereof has been separated from other tag portions by using a digestive enzyme, and has been then purified.

A monoclonal antibody can be easily prepared from an immunized animal according to a publicly-known method of Kohler et al. (Kohler et al., Nature, vol. 256, pp. 495-497 (1975)). Specifically, antibody-producing cells such as spleen cells or lymphocytes are recovered from an immunized animal, and the recovered cells are then fused with mouse myeloma cells according to an ordinary method to produce hybridomas. The obtained hybridomas are cloned by a limiting dilution method, etc., and thereafter, among monoclonal antibodies produced from the cloned hybridomas, a monoclonal antibody having an antigen-antibody reaction with an antigen used in the immunization of the animal is selected.

A monoclonal antibody can be purified from an ascitic fluid or a culture supernatant according to a known immunoglobulin purification method. Examples of the known immunoglobulin purification method may include a fractionation method according to salting-out using ammonium sulfate or sodium sulfate, a PEG fractionation method, an ethanol fractionation method, a DEAE ion exchange chromatography method, and a gel filtration method. Moreover, the monoclonal antibody can also be purified by an affinity chromatography method using a carrier binding to any of Protein A, Protein G, and Protein L, depending on the species of the immunized animal and the class of the monoclonal antibody.

127 Peptides are produced by shifting by 3 amino acids of a partial peptide consisting of 15 amino acids from the amino acid sequence (SEQ ID NO: 128) consisting of 391 amino acids of the N protein of an RS virus, and the reactivity of the produced 127 peptides with the antibody recognizing the N protein of an RS virus of the present invention was then examined. The antibody recognizing the N protein of an RS virus of the present invention reacts with the 30th, 39th, 40th, 69th, 70th, 74th, 75th, 83rd, 84th, 100th, 101st, and 113th peptides among the 127 peptides. The amino acid sequences of the 127 peptides are shown in Figures 1-1 to 1-3. The amino acid sequences of the above-described 1st to 127th peptides (127 peptides) are as set forth in SEQ ID NOs: 1 to 127, respectively.

The antibody recognizing the N protein of an RS virus of the present invention reacts with the following SEQ ID NOs: 30, 39, 40, 69, 70, 74, 75, 83, 84, 100, 101, and 113.
SEQ ID NO: 30: YHVKANGVDVTTHRQ
SEQ ID NO: 39: TLASLTTEIQINIEI
SEQ ID NO: 40: SLTTEIQINIEIESR
SEQ ID NO: 69: IANSFYEVFEKHPHF
SEQ ID NO: 70: SFYEVFEKHPHFIDV
SEQ ID NO: 74: IDVFVHFGIAQSSTR
SEQ ID NO: 75: FVHFGIAQSSTRGGS
SEQ ID NO: 83: FMNAYGAGQVMLRWG
SEQ ID NO: 84: AYGAGQVMLRWGVLA
SEQ ID NO: 100: AGFYHILNNPKASLL
SEQ ID NO: 101: YHILNNPKASLLSLT
SEQ ID NO: 113: YRGTPRNQDLYDAAK

Accordingly, the antibody recognizing the N protein of an RS virus of the present invention recognizes the peptides consisting of the amino acid sequences as set forth in SEQ ID NOs: 30, 39, 40, 69, 70, 74, 75, 83, 84, 100, 101, and 113. It is considered that the antibody recognizing the N protein of an RS virus of the present invention has flexible reactivity, and thus broadly recognizes these peptides. For example, the antibody of the present invention is likely to recognize the three-dimensional structures formed by these peptides.

According to the immunoassay method of the present invention, a measurement is carried out by an immunoassay that utilizes the antigen-antibody reaction of the monoclonal antibody produced as described above or an antigen-binding fragment thereof (hereinafter, in the description until before Examples, the term "antibody" means "an antibody or an antigen-binding fragment thereof," except for the case where it is clearly not the case from the context) with an antigen contained in a specimen. As an immunoassay method applied in the measurement, any of methods publicly known to those skilled in the art, such as a competitive method, an agglutination method, a Western blot method, an immunostaining method, and a sandwich method, can be applied. Besides, in the present invention, the term "measurement" includes any of quantification, semi-quantification, and detection.

The immunoassay is preferably a sandwich method. In the sandwich method, an antigen is sandwiched between two antibodies to form a complex, and the formed complex is detected. The sandwich method itself is publicly known in the field of immunoassay, and this method can be performed, for example, according to an immunochromatography method or an ELISA method. Both of these sandwich methods are publicly known, and the method of the present invention can be carried out according to such a publicly known sandwich method, except for the use of the above-described monoclonal antibody that recognizes the N protein as an antigen.

In the sandwich method, one or two or more types of antibodies that recognize an antigen (an antibody to be immobilized on a solid phase and a labeled antibody) are used. In the case of using two or more types of antibodies, at least any one of these two types of antibodies is the above-described monoclonal antibody that recognizes the N protein as an antigen. Otherwise, an antigen may be sandwiched between antibodies of the same type to form a complex. Preferably, one or two types of antibodies that recognize the above-described peptides are used.

In an immunoassay involving a sandwich method as a detection principle, as a solid phase on which the antibody is immobilized, all of those capable of immobilizing the antibody thereon according to a known technique can be used. For example, a known solid phase, such as a porous thin membrane (membrane) having capillary action, a particulate substance, a test tube, or a resin flat plate, can be arbitrarily selected. In addition, as a substance that labels the antibody, an enzyme, a radioisotope, a fluorescent substance, a luminescent substance, a colored particle, a colloidal particle, etc. can be used. From the viewpoint of, in particular, the simplicity and rapidity of a clinical test, among the aforementioned immunoassay methods using various materials, immunochromatography, which is a lateral flow immunoassay method using a membrane, is preferable.

The present invention also provides an immunoassay apparatus capable of performing a lateral flow immunoassay using the monoclonal antibody recognizing the N protein. The immunoassay apparatus provided by the present invention consists of: a support having a detection region, on which an antibody for capturing a measurement subject (an antigen) (Antibody 1) is immobilized; a label region having a movable labeled antibody (Antibody 2); a sample pad for adding dropwise a specimen; an absorption band for absorbing the spread specimen solution, and a backing sheet for adhering these members to one another. This is the immunoassay apparatus, in which at least either Antibody 1 or Antibody 2 is the monoclonal antibody recognizing the N protein of the present invention. This immunoassay apparatus is also referred to as an "immunochromatography test piece."

The support is a material having an ability to immobilize an antibody for capturing a substance to be detected (an antigen) thereon, and the support also has an ability not to prevent the moving of a liquid in the horizontal direction. Preferably, the support is a porous thin membrane having capillary action, which is capable of transporting a liquid and a component dispersed in the liquid due to absorption. The material used for the support is not particularly limited, and examples of the material of the support may include cellulose, nitrocellulose, cellulose acetate, polyvinylidene difluoride (PVDF), glass fiber, nylon, and polyketone. Among these materials, a thin membrane formed using nitrocellulose is more preferable. A membrane, on which the antibody is immobilized, is referred to as an "antibody-immobilized membrane."

The label region consists of a porous base material comprising a labeled antibody, and as a material used for the base material, commonly used glass fiber, non-woven fabric, etc. can be used. For impregnation with a large amount of labeled antibody, the base material is preferably a pad-shaped material having a thickness of approximately 0.3 mm to 0.6 mm. The porous base material that is impregnated with the labeled antibody and is then dried is also referred to as a "dry pad."

For labeling a labeled antibody, enzymes such as alkaline phosphatase or horseradish peroxidase, metal colloids such as gold colloids, silica particles, cellulose particles, colored polystyrene particles, colored latex particles, etc. are used in many cases. In the case of using metal colloidal particles, or colored particles such as colored polystyrene particles or colored latex particles, since coloration is generated as a result of agglutination of these labeling reagents, this coloration is measured. Particles, on which the antibodies are immobilized, are referred to as "antibody-immobilized particles." The amount of the antibody immobilized is not particularly limited, and it is adequate if the antibody may be present in the label region in an amount of several ng to several tens of µg.

The detection region indicates a partial region of the support, on which an antibody for capturing a substance to be detected (an antigen) is immobilized. In the detection region, at least one region, on which an antibody for capturing an antigen is immobilized, is established. The detection region may be included in the support, and an antibody may be immobilized on the support. The amount of the antibody immobilized is not particularly limited, and it is adequate if the antibody may be immobilized on the detection region in an amount of several ng to several tens of µg.

The sample pad is a site to which a specimen is adding dropwise, and is a porous material. The sample pad is a site located most upstream of the immunoassay apparatus. As a material used for the sample pad, commonly used filter paper, glass fiber, non-woven fabric, etc. can be used. In order to use a large amount of specimen in the immunoassay, the sample pad is preferably a pad-shaped material having a thickness of approximately 0.3 mm to 1 mm. Examples of the specimen may also include a sample or the like that is obtained by allowing the specimen to float in another solution, and a sample prepared using the specimen.

The absorption band is a member for absorbing a component that is supplied to the support and is not involved in the reaction in the detection region. As a material used for the absorption band, a water-retentive filter paper, sponge, etc. consisting of a common natural polymer compound or synthetic polymer compound, etc. can be used. In order to promote the developing of the specimen, a material having a high water absorption rate is preferable.

The backing sheet is a member for adhering all of the aforementioned materials, namely, a support, a sample pad, a label region, an absorption band and the like, to one another, with partial overlapping, and for immobilizing them. The backing sheet is not always necessary, if these materials are disposed and immobilized with optimal intervals. However, for the convenience of production or use, it is generally preferable to use the backing sheet.

The immunoassay apparatus of the present invention may further comprise a control display region (a member). The control display region is a site for showing that the test has been accurately carried out. For example, the control display region is located downstream of the detection region, and emits signals such as coloration, when a specimen sample passes through the detection region and reaches the control display region. On the control display region, a substance that binds to an antibody binding to a labeled carrier may be immobilized, or a reagent, such as a pH indicator whose color is changed when the specimen reaches, may be immobilized. When such a labeled carrier-binding antibody is a mouse monoclonal antibody, an anti-mouse IgG antibody may be used.

The size of the immunoassay apparatus is not limited. For example, the present immunoassay apparatus has a vertical length of several cm to some dozen cm, and a horizontal length of several mm to several cm.

The immunoassay apparatus of the present invention may be placed in a containment cassette. By using this containment cassette (vessel), deterioration of the immunoassay apparatus due to, for example, ultraviolet ray or moisture in the air, can be prevented. Moreover, in the case of using a specimen sample having contamination or infectivity, by using the containment cassette, a tester who performs an assay can be prevented from being contaminated or infected. For example, a resin case having an appropriate size may be used as a containment cassette, and the apparatus of the present invention may be placed in the case. The containment cassette and the immunoassay apparatus placed in the cassette are collectively referred to as an "immunoassay device" in some cases.

An RS virus detection reagent containing the anti-RS virus N protein monoclonal antibody of the present invention comprises the above-described immunoassay apparatus. In addition, an RS virus detection kit containing the anti-RS virus N protein monoclonal antibody of the present invention comprises the above-described immunoassay apparatus. The kit may further comprise a manual, a specimen-collecting device, etc.

According to the method of the present invention, utilizing capillary action, a complex of: Antibody 2, which has been labeled with a suitable labeling substance such as a colored polystyrene particle or a gold colloid (a labeling reagent) capable of binding to a substance to be detected; and the substance to be detected, is developed and moved to a solid-phase support, on which Antibody 1 is immobilized. As a result, a complex consisting of an immobilized substance, a substance to be detected, and a labeling reagent is formed on the solid-phase support, and the signals of the labeling reagent emitted from the complex are then detected (in the case of using a gold colloid, the solid-phase support portion, on which the substance capable of binding to the substance to be detected is immobilized, becomes red), so that the substance to be detected can be detected. This immunoassay method can be carried out at a temperature of 5°C to 35°C, and preferably at room temperature.

Besides, the number of detection regions and the type of a labeled antibody contained in the label region are not limited to one. Antibodies corresponding to a plurality of measurement subjects are used, so that two or more antigens can be detected using a single immunoassay apparatus.

According to the method of the present invention, whether or not a subject is infected with an RS virus can be detected. When an N protein is detected in the sample of a subject, the subject can be determined to be infected with an RS virus.

When the antibody recognizing an N protein of the present invention is used, an RS virus can be specifically recognized. The antibody recognizing an N protein does not recognize other viruses such as, for example, Adenovirus, Coxsackievirus, Echo virus, Herpes simplex virus, Human Metapneumovirus, Influenza virus, Measles virus, Mumps virus, and Parainfluenza virus, and thus, the antibody recognizing an N protein does not falsely detect these viruses.

Moreover, even clinically isolated strains, which cannot be detected by using the antibody that recognizes the F protein of an RS virus, can be detected by using the antibody recognizing the N protein of an RS virus of the present invention.

Examples of the specimen sample used herein may include specimens such as pharyngeal or nasal cavity swab, pharyngeal or nasal cavity lavage fluid, nasal cavity aspirate fluid, saliva, serum, rectal swab, feces, feces suspension, urine, and cornea swab.

### Examples

Hereinafter, the present invention will be more specifically described based on the following examples. However, the following examples are not intended to limit the scope of the present invention.

### Example 1: Production of anti-RS virus N protein monoclonal antibody

### 1. Preparation of RS virus N protein antigen

RS virus-sensitive mammalian cells were infected with the RS virus, and were then cultured for several days. Thereafter, a culture solution of the RS virus-infected cells was inactivated by ultraviolet irradiation, and was then used.

### 2. Production of anti-RS virus N protein monoclonal antibody

BALB/c mice were immunized with the RS virus-inactivated antigen prepared in the above 1., and were then bred for a certain period of time. Thereafter, the iliac lymph node was excised from each mouse. According to such "mouse iliac lymph node method" (Sado Y et al., Acta Histochem. Cytochem. 39: 89-94 (2006)), a plurality of hybridoma cell lines producing anti-RS virus N protein antibodies were obtained.

The thus obtained cell line was intraperitoneally administered into a pristane-treated BALB/c mouse, and approximately 2 weeks after the administration, an antibody-containing ascitic fluid was collected. From the obtained ascitic fluid, IgG was purified by an affinity chromatography method using a Protein A column, and a plurality of purified anti-RS virus N protein monoclonal antibodies (hereinafter referred to as "anti-N protein antibodies" at times) were obtained.

In the following examples, taking into consideration reactivity and specificity, antibodies were selected from the obtained plurality of anti-RS virus N protein monoclonal antibodies, and were then used.

### Example 2: Immunoassay apparatus for measuring RS virus

### 1. Immobilization of anti-RS virus N protein antibody on nitrocellulose membrane

A solution prepared by diluting the anti-N protein antibody produced in Example 1 with a buffer solution, and an anti-mouse IgG antibody were prepared. Thereafter, the anti-N protein antibody was linearly applied onto the sample pad side of a nitrocellulose membrane backed with a PET film, and the anti-mouse IgG antibody was linearly applied onto the absorbent side thereof. Thereafter, the nitrocellulose membrane was fully dried with warm air, so as to obtain an anti-N protein antibody-immobilized membrane.

### 2. Immobilization of anti-RS virus N protein antibody on colored polystyrene particles

The anti-N protein antibody produced in Example 1 was covalently bound to colored polystyrene particles, and the resultant was suspended in a floating liquid and was fully dispersed therein by an ultrasonic treatment, so as to obtain anti-N protein antibody-binding colored polystyrene particles. In the present description, the anti-N protein antibody-binding colored polystyrene particles are referred to as "anti-N protein antibody-immobilized particles."

### 3. Application of anti-RS virus N protein antibody-binding colored polystyrene particles and drying thereof

The antibody-immobilized particles produced in the above 2. were applied in a predetermined amount onto a glass fiber non-woven fabric, and were then fully dried with warm air. In the present description, the thus obtained product is referred to as a "labeling pad."

### 4. Production of RS virus-testing device

The antibody-immobilized membrane produced in the above 1., the labeling pad produced in the above 2. and 3., and other members (a backing sheet, an absorption band, and a sample pad) were adhered to one another, and the obtained product was then cut to a width of 5 mm, thereby obtaining an RS virus-testing device.

### 5. Confirmation of specificity and accuracy of RS virus-testing device

A specimen suspended solution (50 µL) comprising a virus causing respiratory tract infection (10 mM Tris (pH 8.0), 1% (w/v) polyoxyethylene octyl phenyl ether, 3% (w/v) arginine, and 3% (w/v) BSA) was added dropwise onto the RS virus-testing device produced in the above 4., and was then left at rest for 5 minutes.

When coloration could be confirmed by visual observation at the positions, onto which both the anti-mouse IgG antibody and the anti-N protein antibody had been applied, it was determined to be +. When coloration could be confirmed by visual observation only at the position, onto which the anti-mouse IgG antibody had been applied, but the coloration could not be confirmed at the position, onto which the anti-N protein antibody had been applied, it was determined to be -. Moreover, when coloration could not be confirmed by visual observation at the position, onto which the anti-mouse IgG antibody had been applied, it was determined to be invalid.

The results are shown in Table 1.

**[Table 1]**

| Virus name | Measurement results |
|---|---|
| RS virus Long strain (type A) | + |
| RS virus A-2 strain (type A) | + |
| RS virus CH-18 strain (type B) | + |
| Adenovirus type 1 | - |
| Adenovirus type 2 | - |
| Adenovirus type 3 | - |
| Adenovirus type 4 | - |
| Adenovirus type 5 | - |
| Adenovirus type 7 | - |
| Adenovirus type 19 | - |
| Coxsackievirus type A9 | - |
| Coxsackievirus type B4 | - |
| Coxsackievirus type B5 | - |
| Coxsackievirus type B6 | - |
| Echo virus type 2 | - |
| Echo virus type 3 | - |
| Echo virus type 4 | - |
| Echo virus type 6 | - |
| Echo virus type 9 | - |
| Echo virus type 11 | - |
| Echo virus type 30 | - |
| Herpes simplex virus type 1 | - |
| Human Metapneumovirus type A | - |
| Human Metapneumovirus type B | - |
| Influenza virus A/New Caledonia/20/99 (H1N1) | - |
| Influenza virus A/Beijing/262/95 (H1N1) | - |
| Influenza virus A/New York/55/2004 (H3N2) | - |
| Influenza virus A/Hiroshima/52/2005 (H3N2) | - |
| Influenza virus B/Shanghai/361/2002 (Yamagata) | - |
| Influenza virus B/Malaysia/2506/2004 (Victoria) | - |
| Measles virus | - |
| Mumps virus | - |
| Parainfluenza virus type 1 | - |
| Parainfluenza virus type 2 | - |
| Parainfluenza virus type 3 | - |
| Parainfluenza virus type 4 | - |

As shown in Table 1, the immunoassay apparatus using the anti-RS virus N protein antibody of the present invention reacts with the RS virus, but does not exhibit cross-reactivity with other respiratory tract infection-causing viruses. Accordingly, it could be confirmed that the anti-RS virus N protein antibody of the present invention specifically reacts with the RS virus.

### Example 3: Antigen-recognizing sites of anti-RS virus N protein monoclonal antibody

### 1 Analysis using PepSpot(registered trademark) technique

The antigen-recognizing sites of the anti-RS virus N protein monoclonal antibody were analyzed, based on the amino acid sequence of the RS virus N protein registered in GenBank (Accession No. AHC94758.1), using PepSpot(registered trademark) technique of JPT Peptide Technologies (Volmerstrasse 5 (UTZ), 12489 Berlin, Germany). A PepSpot (registered trademark) membrane was prepared by JPT Peptide Technologies. The principle of this method had previously been introduced and explained by the study paper of Kramer et al.(Cell, 91: 799-809 (1997)). The sequences of the peptides immobilized on the membrane are shown in Figure 1-1, 1-2, and 1-3.

### 2. Analysis method using PepSpot (registered trademark) technique

The analysis was carried out in accordance with the protocols provided by JPT Peptide Technologies. An anti-mouse secondary antibody conjugated with peroxidase was used for signal detection according to a standard method.

### 3. Analysis results obtained using PepSpot (registered trademark) technique

Among 127 peptide spots immobilized on the membrane, the anti-RS virus N protein monoclonal antibody obtained in Example 1 reacted with, at least, Peptide Nos. 30, 39, 40, 69, 70, 74, 75, 83, 84, 100, 101, and 113.

The anti-RS virus N protein antibody of the present invention reacts with, at least, SEQ ID NOs: 30, 39, 40, 69, 70, 74, 75, 83, 84, 100, 101, and 113, among the peptide sequences shown in Figure 1.

### Example 4: Comparison regarding sensitivity of anti-RS virus N protein antibody

An antigen detection reagent using the anti-N protein antibody of the present invention was compared with an antigen detection reagent using a commercially available anti-F protein antibody, in terms of detection sensitivity to three RS virus strains, which had been isolated from clinical specimens and had been then cultured.

Three RS virus strains isolated from clinical specimens collected in 2016 were each inoculated into Vero cells and were then allowed to proliferate. A 2-fold dilution series of a culture supernatant of the RS virus-infected cells was prepared using a buffer solution, and a predetermined amount thereof was added to a specimen-floating liquid, so as to prepare a sample.

The results are shown in Table 2.

**[Table 2]**

| **Clinically isolated strain 1** | Virus concentration (TCID₅₀/mL) | | | | | |
|---|---|---|---|---|---|---|
| | 5.6×10⁵ | 2.8×10⁵ | 1.4×10⁵ | 7.0×10⁴ | 3.5×10⁴ | 1.8×10⁴ |
| Anti-N protein antibody | + | + | + | + | + | - |
| Anti-F protein antibody | + | + | + | + | - | - |

| **Clinically isolated strain 2** | Virus concentration (TCID₅₀/mL) | | | | | |
|---|---|---|---|---|---|---|
| | 6.3×10⁶ | 3.2×10⁶ | 1.6×10⁶ | 7.9×10⁵ | 4.0×10⁵ | 2.0×10⁵ |
| Anti-N protein antibody | + | + | + | + | + | - |
| Anti-F protein antibody | + | + | + | + | + | - |
| **Clinically isolated strain 3** | Virus concentration (TCID₅₀/mL) | | | | | |
| | 5.6×10⁴ | 5.6×10³ | 2.8×10³ | 1.4×10³ | 7.0×10² | 3.5×10² |
| Anti-N protein antibody | + | + | + | + | + | - |
| Anti-F protein antibody | - | - | - | - | - | - |

A sensitivity comparison test was carried out using the 3 virus strains isolated from the clinical specimens. As a result, to two out of the three strains, the antigen detection reagent using the anti-N protein antibody exhibited sensitivity that was equivalent to or greater than that of the antigen detection reagent using the anti-F protein antibody.

With regard to the remaining one of the three strains, the antigen detection reagent using the anti-F protein antibody did not react at all, but the antigen detection reagent using the anti-N protein antibody exhibited high detection sensitivity.

### Industrial Applicability

Using the antibody of the present invention, RS virus infection can be specifically detected.

### Sequence Listing Free Text

SEQ ID NOs: 1 to 127 Synthesized
All publications, patents and patent applications cited in the present description are incorporated herein by reference in their entirety.

## Claims

1. An anti-RS virus N protein monoclonal antibody or an antigen-binding fragment thereof, which is **characterized in that** it reacts with peptides consisting of the amino acid sequences as set forth in, at least, SEQ ID NOs: 30, 39, 40, 69, 70, 74, 75, 83, 84, 100, 101, and 113, among 127 peptide spots produced based on the amino acid sequence of the N protein of an RS virus.

2. The anti-RS virus N protein monoclonal antibody or an antigen-binding fragment thereof according to claim 1, wherein the antigen-binding fragment is a peptide fragment selected from the group consisting of Fab, Fab', F(ab')2, a single-chain antibody (scFv), dsFv, a diabody, and a minibody.

3. An RS virus detection reagent, comprising the monoclonal antibody according to claim 1 or 2, or an antigen-binding fragment thereof.

4. The RS virus detection reagent according to claim 3, which is an immunochromatography reagent.

5. An RS virus detection kit, comprising the monoclonal antibody according to claim 1 or 2, or an antigen-binding fragment thereof.

6. A method for detecting an RS virus, using the monoclonal antibody according to claim 1 or 2, or an antigen-binding fragment thereof, according to an immunological detection method.

7. The method according to claim 6, which is an immunochromatography.


SEQUENCE LISTING

<110>  DENKA Company Limited

<120>  An antibody recognizing anti-RS virus N protein, and immunoassay
       method and instrument using the antibody

<130>  PH-8635-PCT

<150>  JP 2019-204604
<151>  2019-11-12

<160>  128

<170>  PatentIn version 3.5

<210>  1
<211>  15
<212>  PRT
<213>  Artificial

<220>
<223>  Synthetic

<400>  1

Met Ala Leu Ser Lys Val Lys Leu Asn Asp Thr Leu Asn Lys Asp
1               5                   10                  15


<210>  2
<211>  15
<212>  PRT
<213>  Artificial

<220>
<223>  Synthetic

<400>  2

Ser Lys Val Lys Leu Asn Asp Thr Leu Asn Lys Asp Gln Leu Leu
1               5                   10                  15


<210>  3
<211>  15
<212>  PRT
<213>  Artificial

<220>
<223>  Synthetic

<400>  3

Lys Leu Asn Asp Thr Leu Asn Lys Asp Gln Leu Leu Ser Ser Ser
1               5                   10                  15


<210>  4
<211>  15
<212>  PRT
<213>  Artificial

<220>
<223>  Synthetic

<400>  4

Asp Thr Leu Asn Lys Asp Gln Leu Leu Ser Ser Ser Lys Tyr Ala
1               5                   10                  15


<210>  5
<211>  15
<212>  PRT
<213>  Artificial

<220>
<223>  Synthetic

<400>  5

Asn Lys Asp Gln Leu Leu Ser Ser Ser Lys Tyr Ala Ile Gln Arg
1               5                   10                  15


<210>  6
<211>  15
<212>  PRT
<213>  Artificial

<220>
<223>  Synthetic

<400>  6

Gln Leu Leu Ser Ser Ser Lys Tyr Ala Ile Gln Arg Ser Thr Gly
1               5                   10                  15


<210>  7
<211>  15
<212>  PRT
<213>  Artificial

<220>
<223>  Synthetic

<400>  7

Ser Ser Ser Lys Tyr Ala Ile Gln Arg Ser Thr Gly Asp Ser Ile
1               5                   10                  15


<210>  8
<211>  15
<212>  PRT
<213>  Artificial

<220>
<223>  Synthetic

<400>  8

Lys Tyr Ala Ile Gln Arg Ser Thr Gly Asp Ser Ile Asp Thr Pro
1               5                   10                  15

<210>  9
<211>  15
<212>  PRT
<213>  Artificial

<220>
<223>  Synthetic

<400>  9

Ile Gln Arg Ser Thr Gly Asp Ser Ile Asp Thr Pro Asn Tyr Asp
1               5                   10                  15


<210>  10
<211>  15
<212>  PRT
<213>  Artificial

<220>
<223>  Synthetic

<400>  10

Ser Thr Gly Asp Ser Ile Asp Thr Pro Asn Tyr Asp Val Gln Lys
1               5                   10                  15


<210>  11
<211>  15
<212>  PRT
<213>  Artificial

<220>
<223>  Synthetic

<400>  11

Asp Ser Ile Asp Thr Pro Asn Tyr Asp Val Gln Lys His Ile Asn
1               5                   10                  15


<210>  12
<211>  15
<212>  PRT
<213>  Artificial

<220>
<223>  Synthetic

<400>  12

Asp Thr Pro Asn Tyr Asp Val Gln Lys His Ile Asn Lys Leu Cys
1               5                   10                  15


<210>  13
<211>  15
<212>  PRT
<213>  Artificial

<220>
<223>  Synthetic

<400>  13

Asn Tyr Asp Val Gln Lys His Ile Asn Lys Leu Cys Gly Met Leu
1               5                   10                  15


<210>  14
<211>  15
<212>  PRT
<213>  Artificial

<220>
<223>  Synthetic

<400>  14

Val Gln Lys His Ile Asn Lys Leu Cys Gly Met Leu Leu Ile Thr
1               5                   10                  15


<210>  15
<211>  15
<212>  PRT
<213>  Artificial

<220>
<223>  Synthetic

<400>  15

His Ile Asn Lys Leu Cys Gly Met Leu Leu Ile Thr Glu Asp Ala
1               5                   10                  15


<210>  16
<211>  15
<212>  PRT
<213>  Artificial

<220>
<223>  Synthetic

<400>  16

Lys Leu Cys Gly Met Leu Leu Ile Thr Glu Asp Ala Asn His Lys
1               5                   10                  15


<210>  17
<211>  15
<212>  PRT
<213>  Artificial

<220>
<223>  Synthetic

<400>  17

Gly Met Leu Leu Ile Thr Glu Asp Ala Asn His Lys Phe Thr Gly
1               5                   10                  15

<210>  18
<211>  15
<212>  PRT
<213>  Artificial

<220>
<223>  Synthetic

<400>  18

Leu Ile Thr Glu Asp Ala Asn His Lys Phe Thr Gly Leu Ile Gly
1               5                   10                  15


<210>  19
<211>  15
<212>  PRT
<213>  Artificial

<220>
<223>  Synthetic

<400>  19

Glu Asp Ala Asn His Lys Phe Thr Gly Leu Ile Gly Met Leu Tyr
1               5                   10                  15


<210>  20
<211>  15
<212>  PRT
<213>  Artificial

<220>
<223>  Synthetic

<400>  20

Asn His Lys Phe Thr Gly Leu Ile Gly Met Leu Tyr Ala Met Ser
1               5                   10                  15


<210>  21
<211>  15
<212>  PRT
<213>  Artificial

<220>
<223>  Synthetic

<400>  21

Phe Thr Gly Leu Ile Gly Met Leu Tyr Ala Met Ser Arg Leu Gly
1               5                   10                  15


<210>  22
<211>  15
<212>  PRT
<213>  Artificial

<220>
<223>  Synthetic

<400>  22

Leu Ile Gly Met Leu Tyr Ala Met Ser Arg Leu Gly Arg Glu Asp
1               5                   10                  15


<210>  23
<211>  15
<212>  PRT
<213>  Artificial

<220>
<223>  Synthetic

<400>  23

Met Leu Tyr Ala Met Ser Arg Leu Gly Arg Glu Asp Thr Ile Lys
1               5                   10                  15


<210>  24
<211>  15
<212>  PRT
<213>  Artificial

<220>
<223>  Synthetic

<400>  24

Ala Met Ser Arg Leu Gly Arg Glu Asp Thr Ile Lys Ile Leu Arg
1               5                   10                  15


<210>  25
<211>  15
<212>  PRT
<213>  Artificial

<220>
<223>  Synthetic

<400>  25

Arg Leu Gly Arg Glu Asp Thr Ile Lys Ile Leu Arg Asp Ala Gly
1               5                   10                  15


<210>  26
<211>  15
<212>  PRT
<213>  Artificial

<220>
<223>  Synthetic

<400>  26

Arg Glu Asp Thr Ile Lys Ile Leu Arg Asp Ala Gly Tyr His Val
1               5                   10                  15

<210>  27
<211>  15
<212>  PRT
<213>  Artificial

<220>
<223>  Synthetic

<400>  27

Thr Ile Lys Ile Leu Arg Asp Ala Gly Tyr His Val Lys Ala Asn
1               5                   10                  15


<210>  28
<211>  15
<212>  PRT
<213>  Artificial

<220>
<223>  Synthetic

<400>  28

Ile Leu Arg Asp Ala Gly Tyr His Val Lys Ala Asn Gly Val Asp
1               5                   10                  15


<210>  29
<211>  15
<212>  PRT
<213>  Artificial

<220>
<223>  Synthetic

<400>  29

Asp Ala Gly Tyr His Val Lys Ala Asn Gly Val Asp Val Thr Thr
1               5                   10                  15


<210>  30
<211>  15
<212>  PRT
<213>  Artificial

<220>
<223>  Synthetic

<400>  30

Tyr His Val Lys Ala Asn Gly Val Asp Val Thr Thr His Arg Gln
1               5                   10                  15


<210>  31
<211>  15
<212>  PRT
<213>  Artificial

<220>
<223>  Synthetic

<400>  31

Lys Ala Asn Gly Val Asp Val Thr Thr His Arg Gln Asp Ile Asn
1               5                   10                  15


<210>  32
<211>  15
<212>  PRT
<213>  Artificial

<220>
<223>  Synthetic

<400>  32

Gly Val Asp Val Thr Thr His Arg Gln Asp Ile Asn Gly Lys Glu
1               5                   10                  15


<210>  33
<211>  15
<212>  PRT
<213>  Artificial

<220>
<223>  Synthetic

<400>  33

Val Thr Thr His Arg Gln Asp Ile Asn Gly Lys Glu Met Lys Phe
1               5                   10                  15


<210>  34
<211>  15
<212>  PRT
<213>  Artificial

<220>
<223>  Synthetic

<400>  34

His Arg Gln Asp Ile Asn Gly Lys Glu Met Lys Phe Glu Val Leu
1               5                   10                  15


<210>  35
<211>  15
<212>  PRT
<213>  Artificial

<220>
<223>  Synthetic

<400>  35

Asp Ile Asn Gly Lys Glu Met Lys Phe Glu Val Leu Thr Leu Ala
1               5                   10                  15

<210>  36
<211>  15
<212>  PRT
<213>  Artificial

<220>
<223>  Synthetic

<400>  36

Gly Lys Glu Met Lys Phe Glu Val Leu Thr Leu Ala Ser Leu Thr
1               5                   10                  15


<210>  37
<211>  15
<212>  PRT
<213>  Artificial

<220>
<223>  Synthetic

<400>  37

Met Lys Phe Glu Val Leu Thr Leu Ala Ser Leu Thr Thr Glu Ile
1               5                   10                  15


<210>  38
<211>  15
<212>  PRT
<213>  Artificial

<220>
<223>  Synthetic

<400>  38

Glu Val Leu Thr Leu Ala Ser Leu Thr Thr Glu Ile Gln Ile Asn
1               5                   10                  15


<210>  39
<211>  15
<212>  PRT
<213>  Artificial

<220>
<223>  Synthetic

<400>  39

Thr Leu Ala Ser Leu Thr Thr Glu Ile Gln Ile Asn Ile Glu Ile
1               5                   10                  15


<210>  40
<211>  15
<212>  PRT
<213>  Artificial

<220>
<223>  Synthetic

<400>  40

Ser Leu Thr Thr Glu Ile Gln Ile Asn Ile Glu Ile Glu Ser Arg
1               5                   10                  15


<210>  41
<211>  15
<212>  PRT
<213>  Artificial

<220>
<223>  Synthetic

<400>  41

Thr Glu Ile Gln Ile Asn Ile Glu Ile Glu Ser Arg Lys Ser Tyr
1               5                   10                  15


<210>  42
<211>  15
<212>  PRT
<213>  Artificial

<220>
<223>  Synthetic

<400>  42

Gln Ile Asn Ile Glu Ile Glu Ser Arg Lys Ser Tyr Lys Lys Met
1               5                   10                  15


<210>  43
<211>  15
<212>  PRT
<213>  Artificial

<220>
<223>  Synthetic

<400>  43

Ile Glu Ile Glu Ser Arg Lys Ser Tyr Lys Lys Met Leu Lys Glu
1               5                   10                  15


<210>  44
<211>  15
<212>  PRT
<213>  Artificial

<220>
<223>  Synthetic

<400>  44

Glu Ser Arg Lys Ser Tyr Lys Lys Met Leu Lys Glu Met Gly Glu
1               5                   10                  15

<210>  45
<211>  15
<212>  PRT
<213>  Artificial

<220>
<223>  Synthetic

<400>  45

Lys Ser Tyr Lys Lys Met Leu Lys Glu Met Gly Glu Val Ala Pro
1               5                   10                  15


<210>  46
<211>  15
<212>  PRT
<213>  Artificial

<220>
<223>  Synthetic

<400>  46

Lys Lys Met Leu Lys Glu Met Gly Glu Val Ala Pro Glu Tyr Arg
1               5                   10                  15


<210>  47
<211>  15
<212>  PRT
<213>  Artificial

<220>
<223>  Synthetic

<400>  47

Leu Lys Glu Met Gly Glu Val Ala Pro Glu Tyr Arg His Asp Ser
1               5                   10                  15


<210>  48
<211>  15
<212>  PRT
<213>  Artificial

<220>
<223>  Synthetic

<400>  48

Met Gly Glu Val Ala Pro Glu Tyr Arg His Asp Ser Pro Asp Cys
1               5                   10                  15


<210>  49
<211>  15
<212>  PRT
<213>  Artificial

<220>
<223>  Synthetic

<400>  49

Val Ala Pro Glu Tyr Arg His Asp Ser Pro Asp Cys Gly Met Ile
1               5                   10                  15


<210>  50
<211>  15
<212>  PRT
<213>  Artificial

<220>
<223>  Synthetic

<400>  50

Glu Tyr Arg His Asp Ser Pro Asp Cys Gly Met Ile Ile Leu Cys
1               5                   10                  15


<210>  51
<211>  15
<212>  PRT
<213>  Artificial

<220>
<223>  Synthetic

<400>  51

His Asp Ser Pro Asp Cys Gly Met Ile Ile Leu Cys Ile Ala Ala
1               5                   10                  15


<210>  52
<211>  15
<212>  PRT
<213>  Artificial

<220>
<223>  Synthetic

<400>  52

Pro Asp Cys Gly Met Ile Ile Leu Cys Ile Ala Ala Leu Val Ile
1               5                   10                  15


<210>  53
<211>  15
<212>  PRT
<213>  Artificial

<220>
<223>  Synthetic

<400>  53

Gly Met Ile Ile Leu Cys Ile Ala Ala Leu Val Ile Thr Lys Leu
1               5                   10                  15

<210>  54
<211>  15
<212>  PRT
<213>  Artificial

<220>
<223>  Synthetic

<400>  54

Ile Leu Cys Ile Ala Ala Leu Val Ile Thr Lys Leu Ala Ala Gly
1               5                   10                  15


<210>  55
<211>  15
<212>  PRT
<213>  Artificial

<220>
<223>  Synthetic

<400>  55

Ile Ala Ala Leu Val Ile Thr Lys Leu Ala Ala Gly Asp Arg Ser
1               5                   10                  15


<210>  56
<211>  15
<212>  PRT
<213>  Artificial

<220>
<223>  Synthetic

<400>  56

Leu Val Ile Thr Lys Leu Ala Ala Gly Asp Arg Ser Gly Leu Thr
1               5                   10                  15


<210>  57
<211>  15
<212>  PRT
<213>  Artificial

<220>
<223>  Synthetic

<400>  57

Thr Lys Leu Ala Ala Gly Asp Arg Ser Gly Leu Thr Ala Val Ile
1               5                   10                  15


<210>  58
<211>  15
<212>  PRT
<213>  Artificial

<220>
<223>  Synthetic

<400>  58

Ala Ala Gly Asp Arg Ser Gly Leu Thr Ala Val Ile Arg Arg Ala
1               5                   10                  15


<210>  59
<211>  15
<212>  PRT
<213>  Artificial

<220>
<223>  Synthetic

<400>  59

Asp Arg Ser Gly Leu Thr Ala Val Ile Arg Arg Ala Asn Asn Val
1               5                   10                  15


<210>  60
<211>  15
<212>  PRT
<213>  Artificial

<220>
<223>  Synthetic

<400>  60

Gly Leu Thr Ala Val Ile Arg Arg Ala Asn Asn Val Leu Lys Asn
1               5                   10                  15


<210>  61
<211>  15
<212>  PRT
<213>  Artificial

<220>
<223>  Synthetic

<400>  61

Ala Val Ile Arg Arg Ala Asn Asn Val Leu Lys Asn Glu Met Lys
1               5                   10                  15


<210>  62
<211>  15
<212>  PRT
<213>  Artificial

<220>
<223>  Synthetic

<400>  62

Arg Arg Ala Asn Asn Val Leu Lys Asn Glu Met Lys Arg Tyr Lys
1               5                   10                  15

<210>  63
<211>  15
<212>  PRT
<213>  Artificial

<220>
<223>  Synthetic

<400>  63

Asn Asn Val Leu Lys Asn Glu Met Lys Arg Tyr Lys Gly Leu Leu
1               5                   10                  15


<210>  64
<211>  15
<212>  PRT
<213>  Artificial

<220>
<223>  Synthetic

<400>  64

Leu Lys Asn Glu Met Lys Arg Tyr Lys Gly Leu Leu Pro Lys Asp
1               5                   10                  15


<210>  65
<211>  15
<212>  PRT
<213>  Artificial

<220>
<223>  Synthetic

<400>  65

Glu Met Lys Arg Tyr Lys Gly Leu Leu Pro Lys Asp Ile Ala Asn
1               5                   10                  15


<210>  66
<211>  15
<212>  PRT
<213>  Artificial

<220>
<223>  Synthetic

<400>  66

Arg Tyr Lys Gly Leu Leu Pro Lys Asp Ile Ala Asn Ser Phe Tyr
1               5                   10                  15


<210>  67
<211>  15
<212>  PRT
<213>  Artificial

<220>
<223>  Synthetic

<400>  67

Gly Leu Leu Pro Lys Asp Ile Ala Asn Ser Phe Tyr Glu Val Phe
1               5                   10                  15


<210>  68
<211>  15
<212>  PRT
<213>  Artificial

<220>
<223>  Synthetic

<400>  68

Pro Lys Asp Ile Ala Asn Ser Phe Tyr Glu Val Phe Glu Lys His
1               5                   10                  15


<210>  69
<211>  15
<212>  PRT
<213>  Artificial

<220>
<223>  Synthetic

<400>  69

Ile Ala Asn Ser Phe Tyr Glu Val Phe Glu Lys His Pro His Phe
1               5                   10                  15


<210>  70
<211>  15
<212>  PRT
<213>  Artificial

<220>
<223>  Synthetic

<400>  70

Ser Phe Tyr Glu Val Phe Glu Lys His Pro His Phe Ile Asp Val
1               5                   10                  15


<210>  71
<211>  15
<212>  PRT
<213>  Artificial

<220>
<223>  Synthetic

<400>  71

Glu Val Phe Glu Lys His Pro His Phe Ile Asp Val Phe Val His
1               5                   10                  15

<210>  72
<211>  15
<212>  PRT
<213>  Artificial

<220>
<223>  Synthetic

<400>  72

Glu Lys His Pro His Phe Ile Asp Val Phe Val His Phe Gly Ile
1               5                   10                  15


<210>  73
<211>  15
<212>  PRT
<213>  Artificial

<220>
<223>  Synthetic

<400>  73

Pro His Phe Ile Asp Val Phe Val His Phe Gly Ile Ala Gln Ser
1               5                   10                  15


<210>  74
<211>  15
<212>  PRT
<213>  Artificial

<220>
<223>  Synthetic

<400>  74

Ile Asp Val Phe Val His Phe Gly Ile Ala Gln Ser Ser Thr Arg
1               5                   10                  15


<210>  75
<211>  15
<212>  PRT
<213>  Artificial

<220>
<223>  Synthetic

<400>  75

Phe Val His Phe Gly Ile Ala Gln Ser Ser Thr Arg Gly Gly Ser
1               5                   10                  15


<210>  76
<211>  15
<212>  PRT
<213>  Artificial

<220>
<223>  Synthetic

<400>  76

Phe Gly Ile Ala Gln Ser Ser Thr Arg Gly Gly Ser Arg Val Glu
1               5                   10                  15


<210>  77
<211>  15
<212>  PRT
<213>  Artificial

<220>
<223>  Synthetic

<400>  77

Ala Gln Ser Ser Thr Arg Gly Gly Ser Arg Val Glu Gly Ile Phe
1               5                   10                  15


<210>  78
<211>  15
<212>  PRT
<213>  Artificial

<220>
<223>  Synthetic

<400>  78

Ser Thr Arg Gly Gly Ser Arg Val Glu Gly Ile Phe Ala Gly Leu
1               5                   10                  15


<210>  79
<211>  15
<212>  PRT
<213>  Artificial

<220>
<223>  Synthetic

<400>  79

Gly Gly Ser Arg Val Glu Gly Ile Phe Ala Gly Leu Phe Met Asn
1               5                   10                  15


<210>  80
<211>  15
<212>  PRT
<213>  Artificial

<220>
<223>  Synthetic

<400>  80

Arg Val Glu Gly Ile Phe Ala Gly Leu Phe Met Asn Ala Tyr Gly
1               5                   10                  15

<210>  81
<211>  15
<212>  PRT
<213>  Artificial

<220>
<223>  Synthetic

<400>  81

Gly Ile Phe Ala Gly Leu Phe Met Asn Ala Tyr Gly Ala Gly Gln
1               5                   10                  15


<210>  82
<211>  15
<212>  PRT
<213>  Artificial

<220>
<223>  Synthetic

<400>  82

Ala Gly Leu Phe Met Asn Ala Tyr Gly Ala Gly Gln Val Met Leu
1               5                   10                  15


<210>  83
<211>  15
<212>  PRT
<213>  Artificial

<220>
<223>  Synthetic

<400>  83

Phe Met Asn Ala Tyr Gly Ala Gly Gln Val Met Leu Arg Trp Gly
1               5                   10                  15


<210>  84
<211>  15
<212>  PRT
<213>  Artificial

<220>
<223>  Synthetic

<400>  84

Ala Tyr Gly Ala Gly Gln Val Met Leu Arg Trp Gly Val Leu Ala
1               5                   10                  15


<210>  85
<211>  15
<212>  PRT
<213>  Artificial

<220>
<223>  Synthetic

<400>  85

Ala Gly Gln Val Met Leu Arg Trp Gly Val Leu Ala Lys Ser Val
1               5                   10                  15


<210>  86
<211>  15
<212>  PRT
<213>  Artificial

<220>
<223>  Synthetic

<400>  86

Val Met Leu Arg Trp Gly Val Leu Ala Lys Ser Val Lys Asn Ile
1               5                   10                  15


<210>  87
<211>  15
<212>  PRT
<213>  Artificial

<220>
<223>  Synthetic

<400>  87

Arg Trp Gly Val Leu Ala Lys Ser Val Lys Asn Ile Met Leu Gly
1               5                   10                  15


<210>  88
<211>  15
<212>  PRT
<213>  Artificial

<220>
<223>  Synthetic

<400>  88

Val Leu Ala Lys Ser Val Lys Asn Ile Met Leu Gly His Ala Ser
1               5                   10                  15


<210>  89
<211>  15
<212>  PRT
<213>  Artificial

<220>
<223>  Synthetic

<400>  89

Lys Ser Val Lys Asn Ile Met Leu Gly His Ala Ser Val Gln Ala
1               5                   10                  15

<210>  90
<211>  15
<212>  PRT
<213>  Artificial

<220>
<223>  Synthetic

<400>  90

Lys Asn Ile Met Leu Gly His Ala Ser Val Gln Ala Glu Met Glu
1               5                   10                  15


<210>  91
<211>  15
<212>  PRT
<213>  Artificial

<220>
<223>  Synthetic

<400>  91

Met Leu Gly His Ala Ser Val Gln Ala Glu Met Glu Gln Val Val
1               5                   10                  15


<210>  92
<211>  15
<212>  PRT
<213>  Artificial

<220>
<223>  Synthetic

<400>  92

His Ala Ser Val Gln Ala Glu Met Glu Gln Val Val Glu Val Tyr
1               5                   10                  15


<210>  93
<211>  15
<212>  PRT
<213>  Artificial

<220>
<223>  Synthetic

<400>  93

Val Gln Ala Glu Met Glu Gln Val Val Glu Val Tyr Glu Tyr Ala
1               5                   10                  15


<210>  94
<211>  15
<212>  PRT
<213>  Artificial

<220>
<223>  Synthetic

<400>  94

Glu Met Glu Gln Val Val Glu Val Tyr Glu Tyr Ala Gln Lys Leu
1               5                   10                  15


<210>  95
<211>  15
<212>  PRT
<213>  Artificial

<220>
<223>  Synthetic

<400>  95

Gln Val Val Glu Val Tyr Glu Tyr Ala Gln Lys Leu Gly Gly Glu
1               5                   10                  15


<210>  96
<211>  15
<212>  PRT
<213>  Artificial

<220>
<223>  Synthetic

<400>  96

Glu Val Tyr Glu Tyr Ala Gln Lys Leu Gly Gly Glu Ala Gly Phe
1               5                   10                  15


<210>  97
<211>  15
<212>  PRT
<213>  Artificial

<220>
<223>  Synthetic

<400>  97

Glu Tyr Ala Gln Lys Leu Gly Gly Glu Ala Gly Phe Tyr His Ile
1               5                   10                  15


<210>  98
<211>  15
<212>  PRT
<213>  Artificial

<220>
<223>  Synthetic

<400>  98

Gln Lys Leu Gly Gly Glu Ala Gly Phe Tyr His Ile Leu Asn Asn
1               5                   10                  15

<210>  99
<211>  15
<212>  PRT
<213>  Artificial

<220>
<223>  Synthetic

<400>  99

Gly Gly Glu Ala Gly Phe Tyr His Ile Leu Asn Asn Pro Lys Ala
1               5                   10                  15


<210>  100
<211>  15
<212>  PRT
<213>  Artificial

<220>
<223>  Synthetic

<400>  100

Ala Gly Phe Tyr His Ile Leu Asn Asn Pro Lys Ala Ser Leu Leu
1               5                   10                  15


<210>  101
<211>  15
<212>  PRT
<213>  Artificial

<220>
<223>  Synthetic

<400>  101

Tyr His Ile Leu Asn Asn Pro Lys Ala Ser Leu Leu Ser Leu Thr
1               5                   10                  15


<210>  102
<211>  15
<212>  PRT
<213>  Artificial

<220>
<223>  Synthetic

<400>  102

Leu Asn Asn Pro Lys Ala Ser Leu Leu Ser Leu Thr Gln Phe Pro
1               5                   10                  15


<210>  103
<211>  15
<212>  PRT
<213>  Artificial

<220>
<223>  Synthetic

<400>  103

Pro Lys Ala Ser Leu Leu Ser Leu Thr Gln Phe Pro His Phe Ser
1               5                   10                  15


<210>  104
<211>  15
<212>  PRT
<213>  Artificial

<220>
<223>  Synthetic

<400>  104

Ser Leu Leu Ser Leu Thr Gln Phe Pro His Phe Ser Ser Val Val
1               5                   10                  15


<210>  105
<211>  15
<212>  PRT
<213>  Artificial

<220>
<223>  Synthetic

<400>  105

Ser Leu Thr Gln Phe Pro His Phe Ser Ser Val Val Leu Gly Asn
1               5                   10                  15


<210>  106
<211>  15
<212>  PRT
<213>  Artificial

<220>
<223>  Synthetic

<400>  106

Gln Phe Pro His Phe Ser Ser Val Val Leu Gly Asn Ala Ala Gly
1               5                   10                  15


<210>  107
<211>  15
<212>  PRT
<213>  Artificial

<220>
<223>  Synthetic

<400>  107

His Phe Ser Ser Val Val Leu Gly Asn Ala Ala Gly Leu Gly Ile
1               5                   10                  15

<210>  108
<211>  15
<212>  PRT
<213>  Artificial

<220>
<223>  Synthetic

<400>  108

Ser Val Val Leu Gly Asn Ala Ala Gly Leu Gly Ile Met Gly Glu
1               5                   10                  15


<210>  109
<211>  15
<212>  PRT
<213>  Artificial

<220>
<223>  Synthetic

<400>  109

Leu Gly Asn Ala Ala Gly Leu Gly Ile Met Gly Glu Tyr Arg Gly
1               5                   10                  15


<210>  110
<211>  15
<212>  PRT
<213>  Artificial

<220>
<223>  Synthetic

<400>  110

Ala Ala Gly Leu Gly Ile Met Gly Glu Tyr Arg Gly Thr Pro Arg
1               5                   10                  15


<210>  111
<211>  15
<212>  PRT
<213>  Artificial

<220>
<223>  Synthetic

<400>  111

Leu Gly Ile Met Gly Glu Tyr Arg Gly Thr Pro Arg Asn Gln Asp
1               5                   10                  15


<210>  112
<211>  15
<212>  PRT
<213>  Artificial

<220>
<223>  Synthetic

<400>  112

Met Gly Glu Tyr Arg Gly Thr Pro Arg Asn Gln Asp Leu Tyr Asp
1               5                   10                  15


<210>  113
<211>  15
<212>  PRT
<213>  Artificial

<220>
<223>  Synthetic

<400>  113

Tyr Arg Gly Thr Pro Arg Asn Gln Asp Leu Tyr Asp Ala Ala Lys
1               5                   10                  15


<210>  114
<211>  15
<212>  PRT
<213>  Artificial

<220>
<223>  Synthetic

<400>  114

Thr Pro Arg Asn Gln Asp Leu Tyr Asp Ala Ala Lys Ala Tyr Ala
1               5                   10                  15


<210>  115
<211>  15
<212>  PRT
<213>  Artificial

<220>
<223>  Synthetic

<400>  115

Asn Gln Asp Leu Tyr Asp Ala Ala Lys Ala Tyr Ala Glu Gln Leu
1               5                   10                  15


<210>  116
<211>  15
<212>  PRT
<213>  Artificial

<220>
<223>  Synthetic

<400>  116

Leu Tyr Asp Ala Ala Lys Ala Tyr Ala Glu Gln Leu Lys Glu Asn
1               5                   10                  15

<210>  117
<211>  15
<212>  PRT
<213>  Artificial

<220>
<223>  Synthetic

<400>  117

Ala Ala Lys Ala Tyr Ala Glu Gln Leu Lys Glu Asn Gly Val Ile
1               5                   10                  15


<210>  118
<211>  15
<212>  PRT
<213>  Artificial

<220>
<223>  Synthetic

<400>  118

Ala Tyr Ala Glu Gln Leu Lys Glu Asn Gly Val Ile Asn Tyr Ser
1               5                   10                  15


<210>  119
<211>  15
<212>  PRT
<213>  Artificial

<220>
<223>  Synthetic

<400>  119

Glu Gln Leu Lys Glu Asn Gly Val Ile Asn Tyr Ser Val Leu Asp
1               5                   10                  15


<210>  120
<211>  15
<212>  PRT
<213>  Artificial

<220>
<223>  Synthetic

<400>  120

Lys Glu Asn Gly Val Ile Asn Tyr Ser Val Leu Asp Leu Thr Ala
1               5                   10                  15


<210>  121
<211>  15
<212>  PRT
<213>  Artificial

<220>
<223>  Synthetic

<400>  121

Gly Val Ile Asn Tyr Ser Val Leu Asp Leu Thr Ala Glu Glu Leu
1               5                   10                  15


<210>  122
<211>  15
<212>  PRT
<213>  Artificial

<220>
<223>  Synthetic

<400>  122

Asn Tyr Ser Val Leu Asp Leu Thr Ala Glu Glu Leu Glu Ala Ile
1               5                   10                  15


<210>  123
<211>  15
<212>  PRT
<213>  Artificial

<220>
<223>  Synthetic

<400>  123

Val Leu Asp Leu Thr Ala Glu Glu Leu Glu Ala Ile Lys His Gln
1               5                   10                  15


<210>  124
<211>  15
<212>  PRT
<213>  Artificial

<220>
<223>  Synthetic

<400>  124

Leu Thr Ala Glu Glu Leu Glu Ala Ile Lys His Gln Leu Asn Pro
1               5                   10                  15


<210>  125
<211>  15
<212>  PRT
<213>  Artificial

<220>
<223>  Synthetic

<400>  125

Glu Glu Leu Glu Ala Ile Lys His Gln Leu Asn Pro Lys Asp Asn
1               5                   10                  15

<210>  126
<211>  15
<212>  PRT
<213>  Artificial

<220>
<223>  Synthetic

<400>  126

Glu Ala Ile Lys His Gln Leu Asn Pro Lys Asp Asn Asp Val Glu
1               5                   10                  15


<210>  127
<211>  15
<212>  PRT
<213>  Artificial

<220>
<223>  Synthetic

<400>  127

Ala Ile Lys His Gln Leu Asn Pro Lys Asp Asn Asp Val Glu Leu
1               5                   10                  15


<210>  128
<211>  391
<212>  PRT
<213>  Human respiratory syncytial virus

<400>  128

Met Ala Leu Ser Lys Val Lys Leu Asn Asp Thr Leu Asn Lys Asp Gln
1               5                   10                  15


Leu Leu Ser Ser Ser Lys Tyr Ala Ile Gln Arg Ser Thr Gly Asp Ser
            20                  25                  30


Ile Asp Thr Pro Asn Tyr Asp Val Gln Lys His Ile Asn Lys Leu Cys
        35                  40                  45


Gly Met Leu Leu Ile Thr Glu Asp Ala Asn His Lys Phe Thr Gly Leu
    50                  55                  60


Ile Gly Met Leu Tyr Ala Met Ser Arg Leu Gly Arg Glu Asp Thr Ile
65                  70                  75                  80


Lys Ile Leu Arg Asp Ala Gly Tyr His Val Lys Ala Asn Gly Val Asp
                85                  90                  95


Val Thr Thr His Arg Gln Asp Ile Asn Gly Lys Glu Met Lys Phe Glu
            100                 105                 110

Val Leu Thr Leu Ala Ser Leu Thr Thr Glu Ile Gln Ile Asn Ile Glu
        115                 120                 125

Ile Glu Ser Arg Lys Ser Tyr Lys Lys Met Leu Lys Glu Met Gly Glu
    130                 135                 140

Val Ala Pro Glu Tyr Arg His Asp Ser Pro Asp Cys Gly Met Ile Ile
145                 150                 155                 160

Leu Cys Ile Ala Ala Leu Val Ile Thr Lys Leu Ala Ala Gly Asp Arg
                165                 170                 175

Ser Gly Leu Thr Ala Val Ile Arg Arg Ala Asn Asn Val Leu Lys Asn
            180                 185                 190

Glu Met Lys Arg Tyr Lys Gly Leu Leu Pro Lys Asp Ile Ala Asn Ser
        195                 200                 205

Phe Tyr Glu Val Phe Glu Lys His Pro His Phe Ile Asp Val Phe Val
    210                 215                 220

His Phe Gly Ile Ala Gln Ser Ser Thr Arg Gly Gly Ser Arg Val Glu
225                 230                 235                 240

Gly Ile Phe Ala Gly Leu Phe Met Asn Ala Tyr Gly Ala Gly Gln Val
                245                 250                 255

Met Leu Arg Trp Gly Val Leu Ala Lys Ser Val Lys Asn Ile Met Leu
            260                 265                 270

Gly His Ala Ser Val Gln Ala Glu Met Glu Gln Val Val Glu Val Tyr
        275                 280                 285

Glu Tyr Ala Gln Lys Leu Gly Gly Glu Ala Gly Phe Tyr His Ile Leu
    290                 295                 300

Asn Asn Pro Lys Ala Ser Leu Leu Ser Leu Thr Gln Phe Pro His Phe
305                 310                 315                 320

Ser Ser Val Val Leu Gly Asn Ala Ala Gly Leu Gly Ile Met Gly Glu
                325                 330                 335

Tyr Arg Gly Thr Pro Arg Asn Gln Asp Leu Tyr Asp Ala Ala Lys Ala
            340                 345                 350

Tyr Ala Glu Gln Leu Lys Glu Asn Gly Val Ile Asn Tyr Ser Val Leu

        355                 360                 365

Asp Leu Thr Ala Glu Glu Leu Glu Ala Ile Lys His Gln Leu Asn Pro
    370                 375                 380

Lys Asp Asn Asp Val Glu Leu
385                 390